# EUROPEAN PATENT APPLICATION

(11) **EP 3 778 849 A1**
(43) Date of publication of application: **17.02.2021**
(21) Application number: 18913172.5
(22) Date of filing: 28.08.2018
(51) Int. Cl.: C12M 1/00, C12N 5/10

(54) **CLEAN SPACE MAINTENANCE DEVICE, BUILDING STRUCTURE, CELL CULTURING METHOD, AND CELL PRODUCTION METHOD**

(30) Priority: 26.03.2018 JP 2018058670
(71) Applicant: Sharp Kabushiki Kaisha, Sakai-shi Osaka 590-8522 (JP)
(72) Inventor: YAMAMOTO, Satohiko, Osaka 590-8522 (JP); UENISHI, Akira, Osaka 590-8522 (JP)
(74) Representative: Treeby, Philip David William
(86) International application number: PCT/JP2018/031830
(87) International publication number: WO 2019/187201

(57) **Abstract**

Provided are such features as a cell culturing method capable of continuously culturing cells. A workroom (40) includes an ion generator (47) supplying positive and negative ions into a work space (48).

## Description

### Technical Field

The present invention relates to a cell culturing method in a clean space, and a device and a facility for the cell culturing method.

### Background Art

Work on culturing cells is conducted in a clean space (a sterilized space) to prevent contamination of the cells. Patent Document 1 discloses an example of a safety cabinet to define a clean space.

### Citation List

### Patent Literature

[Patent Document 1] Japanese Unexamined Patent Application Publication No. 2016-165249

### Summary of Invention

### Technical Problem

In order to maintain a clean space, the clean space needs to be sterilized whenever appropriate while cells are cultured. Sometimes, a whole research facility would be sterilized. Typical sterilization is carried out, using formalin, a peracetic acid product, hydrogen peroxide, or ultraviolet.

Unfortunately, during such typical sterilization (approximately several hours to one week), cells cannot be cultured and the work on the cell culturing cannot be conducted.

An aspect of the present invention intends to provide a clean space maintenance device and a cell culturing method which allow for continuous cell culturing or continuous work on the cell culturing.

### Solution to Problem

In order to overcome the above problem, a clean space maintenance device according to an embodiment of the present invention includes: a space definer defining a space for culturing cells or conducting work on the culturing; and an inhibitor supplier supplying a first microorganism growth inhibitor to the space, or a second microorganism growth inhibitor to a filter allowing passage of gas to be supplied to the space, the first microorganism growth inhibitor causing no harm to humans.

A cell culturing method according to an embodiment of the present invention includes steps of: (a) supplying a first microorganism growth inhibitor to a space for culturing cells or conducting work on the culturing, or clean air to the space, the first microorganism growth inhibitor causing no harm to humans, and the clean air being generated using a second microorganism growth inhibitor; and (b) culturing cells or conducting work on the culturing inside the space supplied with the first microorganism growth inhibitor, or with the clean air.

A cell production method according to an embodiment of the present invention includes steps of: (a) supplying a first microorganism growth inhibitor to a space for culturing cells or conducting work on the culturing, or clean air to the space, the first microorganism growth inhibitor causing no harm to humans, and the clean air being generated using a second microorganism growth inhibitor; and (b) culturing cells or conducting work on the culturing inside the space supplied with the first microorganism growth inhibitor, or with the clean air, thereby producing cultured cells.

A building structure according to an embodiment of the present invention defines a space for culturing cells or conducting work on the culturing. The building structure includes an inhibitor supplier supplying a first microorganism growth inhibitor to the space, or a second microorganism growth inhibitor to a filter allowing passage of gas to be supplied to the space, the first microorganism growth inhibitor causing no harm to humans.

### Advantageous Effects of Invention

An aspect of the present invention allows for continuous cell culturing or continuous work on the cell culturing without interruption for sterilization.

### Brief Description of Drawings

[FIG. 1] FIG. 1 is a cross-sectional view illustrating a configuration of a safety cabinet according to a first embodiment.
[FIG. 2] FIG. 2 is a diagram illustrating a configuration of an ion generator.
[FIG. 3] FIG. 3 is a cross-sectional view illustrating a configuration of a clean chamber according to a second embodiment.
[FIG. 4] FIG. 4 is a cross-sectional view of an incubator according to a third embodiment, the cross-sectional view being illustrated on a plane in parallel with a front of the incubator.
[FIG. 5] FIG. 5 is a layout illustrating a configuration of a cell culturing system according to a fourth embodiment.
[FIG. 6] FIG. 6 is a diagram illustrating a positional relationship between an inhibitor supplier and a filter in a filtering state.
[FIG. 7] FIG. 7 is a graph illustrating advantageous effects of positive and negative ions on mycoplasma.
[FIG. 8] FIG. 8 is a graph illustrating advantageous effects of positive and negative ions on airborne bacteria.

### Description of Embodiments

### First Embodiment

Described below in detail is an embodiment of the present invention. In this DESCRIPTION, the concept of sterilization includes not only killing microorganisms such as bacteria, molds, and viruses, but also curbing growth and proliferation of the microorganisms and physically removing the microorganisms with a filter. Hereinafter, the microorganisms are also referred to as bacteria.

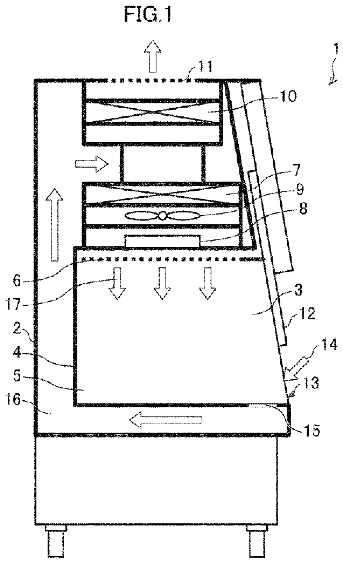

FIG. 1 is a cross-sectional view illustrating a configuration of a safety cabinet 1 according to this embodiment. The safety cabinet 1 is a clean space maintenance device to maintain a clean space (a sterilized space) for conducting the work on cell culturing. The safety cabinet 1 is designed on the assumption of culturing cells of a multicellular organism (e.g., a human) for, in particular, regenerative medicine. An example of the cells to be cultured includes induced pluripotent stem cells (iPS cells). Note that the safety cabinet 1 may be used for asepsis (e.g., culturing bacteria, fungi, or plant cells) other than culturing cells for regenerative medicine.

As illustrated in FIG. 1, the safety cabinet 1 includes a space definer 4 to define a work space 3 for conducting work on culturing cells. The space definer 4 has a bottom face to be used as a workbench 5. A front shutter 12 can also be interpreted as a part of the space definer 4.

Indoor air 14 is sucked from an opening 13 which is a clearance between the front shutter 12 and the workbench 5. The sucked indoor air 14 flows into an opening 15 formed on the workbench 5, passes through a circulation flow passage 16 formed inside a body 12, and is then sucked into an air blower 9. The air sucked into the air blower 9 is filtered with an air supply high efficiency particulate air filter (HEPA) 7, and then passes through an ion generator 8 (an inhibitor supplier) so that the air is provided with positive ions and negative ions (a first microorganism growth inhibitor). The air provided with the positive ions and the negative ions is supplied as clean air 17 (clean gas) into the work space 3.

The ion generator 8 supplies the positive ions and the negative ions so that a positive ion concentration of the positive ions and a negative ion concentration of the negative ions each range preferably from 7,000 ions/cm³ to 1,000,000 ions/cm³, and more preferably from 7,000 ions/cm³ to 400,000 ions/cm³. The positive ion concentration and the negative ion concentration of 7,000 ions/cm³ or higher make it possible to curb growth and proliferation of bacteria. Moreover, the positive ion concentration and the negative ion concentration of 1,000,000 ions/cm³ or lower make it possible to reduce adverse effects on cells to be cultivated. Furthermore, the positive ion concentration and the negative ion concentration of 400,000 ions/cm³ or lower make it possible to reduce the adverse effects more reliably. The inventors of the present invention have confirmed that, when the positive ion concentration and the negative ion concentration are decreased to 400,000 ions/cm³ or lower, the cultured iPS cells are the same in positive rate, viability, and cell proliferation rate as a control. Hereinafter, the positive ions and the negative ions are also collectively referred to as positive and negative ions.

Hence, the ion generator 8, which is installed in the safety cabinet 1, provides the positive and negative ions acting as the first microorganism growth inhibitor to the gas to be supplied to the work space 3. The clean air 17 contains the positive and negative ions, making it possible to curb growth and proliferation of bacteria found inside the work space 3.

Portion of the clean air 17 supplied into the work space 3 flows into the opening 15. The air in the opening 15 is sucked into the air blower 9, and provided with the positive and negative ions again. Hence, the air circulating inside the safety cabinet 1 is continuously provided with the positive and negative ions, maintaining the work space 3 clean.

Meanwhile, portion of the air rising in the circulation flow passage 16 is filtered with an exhaust HEPA 10, and ejected as clean air from an ejection port 11 out of the device.

In this DESCRIPTION, a microorganism growth inhibitor is a substance capable of curbing growth and proliferation of such microorganisms as bacteria and molds causing adverse effects on cell cultivation, a substance capable of deactivating such microorganisms as viruses, or a substance to be used for removing the above microorganisms in the air. The microorganism growth inhibitor is likely to be used in such states as: supplying the microorganism growth inhibitor to the work space 3 (a release state); and supplying the microorganism growth inhibitor to a filter allowing for passage of the gas to be supplied into the work space 3 (a filtering state).

The first microorganism growth inhibitor to be used in the release state is capable of curbing growth and proliferation of bacteria or molds, or of deactivating viruses. Preferably, the first microorganism growth inhibitor neither causes any harm to humans nor substantially inhibit proliferation of cells of multicellular organisms.

Examples of the first microorganism growth inhibitor to be used in the release state include positive and negative ions, and charged microparticles in nanometer level including radical. Examples of a second microorganism growth inhibitor to be used in the filtering state include electric charges, low-temperature plasma, and chlorine compounds. Microorganism growth inhibitors other than the positive and negative ions will be described later in detail.

The air supply HEPA 7 captures microorganisms such as bacteria and viruses, or objects to be deactivated. If the positive and negative ions are sufficiently effective in curbing growth and proliferation of the microorganisms, the air supply HEPA 7 can be omitted.

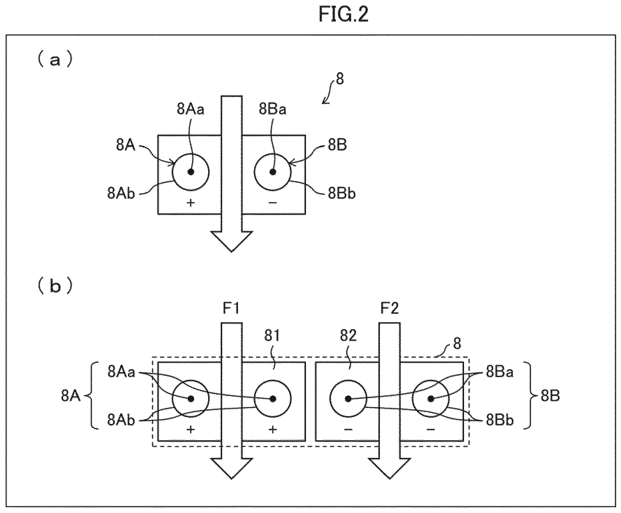

FIG. 2 is a diagram illustrating a configuration of the ion generator 8. As shown in an illustration (a) in FIG. 2, the ion generator 8 includes two ion generation units 8A and 8B arranged in parallel and perpendicularly to airflows F1 and F2. A not-shown power supply supplies a voltage to the ion generation units 8A and 8B, so that the ion generation units 8A and 8B cause a corona discharge to generate ions. The ion generation unit 8A generates positive ions, and the ion generation unit 8B generates negative ions.

Moreover, as shown in the illustration (b) in FIG. 2, the ion generator 8 may include a first unit 81 and a second unit 82 arranged in parallel. The first unit 81 includes two or more ion generation units 8A arranged in parallel, and the second unit 82 includes two or more ion generation units 8B arranged in parallel.

The ion generating units 8A and 8B respectively include: discharge electrode protrusions 8Aa and 8Ba having an end pointed; and induction electrode rings 8Ab and 8Bb respectively surrounding the discharge electrode protrusions 8Aa and 8Ba. The discharge electrode protrusion 8Aa is disposed in a center of the induction electrode ring 8Ab, and the discharge electrode protrusion 8Ba is disposed in a center of the induction electrode ring 8Bb.

Note that each of the discharge electrode protrusions 8Aa and 8Ba may include such an electrode as a flat electrode, a needle electrode, or a brush electrode. The discharge electrode protrusions 8Aa and 8Ba may be of any given shape and kind, or may be made of any given material as long as they are dischargeable.

Applied to the ion generation unit 8A is a positive voltage. When the positive voltage is applied to the ion generation unit 8A, water molecules in the air are electrolyzed in a plasma region caused by the electric discharge, thereby mainly generating hydrogen ions H⁺. Then, water molecules in the air cohere around the generated hydrogen ions H⁺, thereby generating cluster ions H⁺(H₂O)ₘ having positive stable electrical charges.

Applied to the ion generation unit 8B is a negative voltage. When the negative voltage is applied to the ion generation unit 8B, oxygen molecules in the air are electrolyzed in a plasma region caused by the electric discharge, thereby mainly generating oxygen ions O₂⁻. Then, water molecules in the air cohere around the generated oxygen ions O₂⁻, thereby generating cluster ions O₂⁻(H₂O)ₙ having negative stable electrical charges. Here, "m" and "n" are any given integers. In this DESCRIPTION, the term "positive ions" means positive cluster ions, and the term "negative ions" means negative cluster ions. Note that generations of the positive and negative cluster ions can be confirmed by the time-of-flight mass spectrometry.

### Advantageous Effects of Safety Cabinet 1

When simultaneously released in the air, the positive and negative ions cohere on the surfaces of, and surround, airborne microorganisms. Then, the positive ions and the negative ions momentarily combine together, such that [·OH] (hydroxyl radical) or H₂O₂ (hydrogen peroxide) are generated cohesively on the surfaces of the microorganisms. Both [·OH] and H₂O₂, which are significantly oxidative active species, decompose protein on the surfaces of the microorganisms by chemical reaction to curb the action of the microorganisms.

Hence, when the ion generator 8 generates the positive ions and the negative ions to supply the ions to the work space 3, the supplied ions can deactivate the viruses present in the work space 3, and curb growth and proliferation of bacteria and molds found inside the work space 3.

The positive and negative ions cause no harm to humans and cultured cells, making it possible to sterilize the work space 3 while conducting work on cell culturing. Hence, the work on the cell culturing is not interrupted for the sterilization. Such a feature makes it possible to culture the cells efficiently.

A conventional safety cabinet could capture bacteria with the air supply HEPA 7; however, it is difficult for the conventional safety cabinet to curb growth and proliferation of bacteria entering the work space 3.

### Second Embodiment

Described below is another embodiment of the present invention. Note that, for convenience of description, identical constituent features have the same reference signs between this embodiment and the above embodiment. Such constituent features will not be repeatedly elaborated upon.

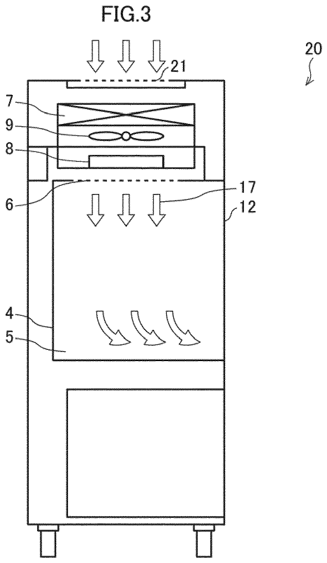

FIG. 3 is a cross-sectional view illustrating a configuration of a clean chamber 20 according to this embodiment. The clean chamber 20 is a clean space maintenance device including therein the ion generator 8. As illustrated in FIG. 3, the clean chamber 20 causes the air blower 9 to supply air from an air supply opening 21, and introduces the supplied air to the air supply HEPA 7. Moreover, the clean chamber 20 causes the ion generator 8 to provide positive and negative ions to the air passing through the air supply HEPA 7. The air provided with the positive and negative ions (the clean air 17) is supplied into the work space 3.

The ion generator 8 supplies the positive ions and the negative ions into the work space 3 so that each of the positive ion concentration and the negative ion concentration ranges preferably from 7,000 ions/cm³ to 1,000,000 ions/cm³, and more preferably from 7,000 ions/cm³ to 400,000 ions/cm³.

The positive ions and negative ions included in the clean air 17 deactivate the viruses present in the work space 3, making it possible to curb growth and proliferation of bacteria and molds found inside the work space 3. The clean chamber 20 can be used as a clean space maintenance device for work on culturing cells for regenerative medicine, and also for other asepsis (e.g., culturing bacteria, fungi, or plant cells).

### Third Embodiment

Described below is still another embodiment of the present invention. Note that, for convenience of description, identical constituent features have the same reference signs between this embodiment and the above embodiments. Such constituent features will not be repeatedly elaborated upon.

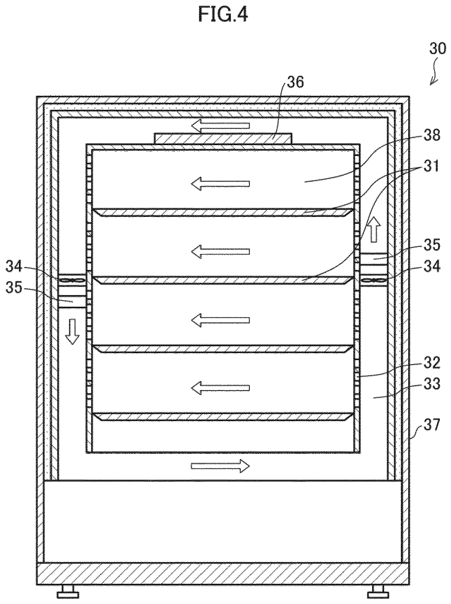

FIG. 4 is a cross-sectional view illustrating a configuration of an incubator 30 (a temperature control device) according to this embodiment. FIG. 4 is a cross-sectional view illustrated on a plane in parallel with a front of the incubator 30. The incubator 30 is a clean space maintenance device including therein an ion generator 35 (an inhibitor supplier).

As illustrated in FIG. 4, the incubator 30 includes: a plurality of shelves 31 on which, for example, a petri dish is placed; a frame 32 holding the shelves 31; an air blower 34; the ion generator 35; and a heater 36. Similar to the ion generator 8, the ion generator 35 generates positive and negative ions.

The ion generator 35 supplies the positive and negative ions so that each of the positive ion concentration and the negative ion concentration ranges preferably from 7,000 ions/cm³ to 1,000,000 ions/cm³, and more preferably from 7,000 ions/cm³ to 400,000 ions/cm³.

The incubator 30 includes a body 37 (a temperature-controlled space definer) defining a culturing space 38 (a temperature-controlled space) for culturing cells. The temperature in this culturing space is adjusted by the heater 36 to an appropriate one for the cell culturing. Furthermore, the body 37 may be provided with a not-shown supply tube for supplying the culturing space 38 with CO₂.

The air blower 34 generates an airflow inside the incubator 30. This airflow circulates through a flow passage 33, flows through a plurality of openings formed on the frame 32, and passes between the shelves 31. In the flow passage 33, the ion generator 35 is positioned more downstream of the airflow than the air blower 34 is. Hence, the positive and negative ions generated by the ion generator 35 can circulate inside the incubator 30, and maintain the inside of the incubator 30 clean.

### Fourth Embodiment

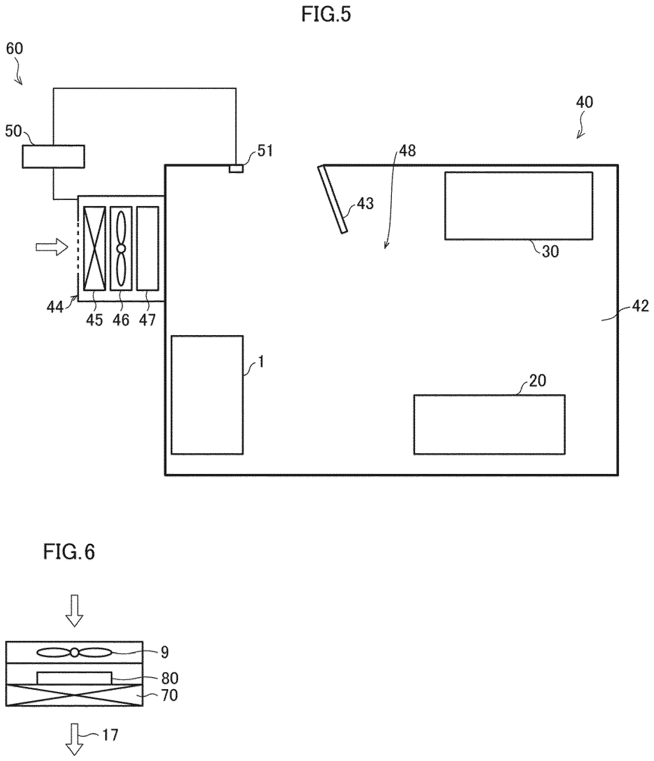

Described below is still another embodiment of the present invention. FIG. 5 is a layout illustrating a configuration of a cell culturing system 60 according to this embodiment. The cell culturing system 60 (a cell culturing facility) is for culturing cells of a multicellular organism for regenerative medicine. Note that the cell culturing system 60 may also be used for culturing cells for other than those for regenerative medicine.

The cell culturing system 60 includes: a workroom 40 (a building structure); and a control device 50. The cell culturing system 60 may include a plurality of workrooms 40. The workroom 40 includes: a plurality of wall faces 41; a floor 42; and a not-shown ceiling all of which define a work space 48. The work space 48 is for culturing cells or for conducting work on the cell culturing. At least one of the wall faces 41 is provided with at least one door 43. The door 43 is opened and closed to allow a worker to enter and exit the work space 48.

In the work space 48, the safety cabinet 1, the clean chamber 20, and the incubator 30 are arranged. The arrangement of these devices is not limited to any particular one. Not all of these devices have to be arranged. Instead of these devices, a conventional safety cabinet, a conventional clean chamber, or a conventional incubator may be disposed.

At least either the ceiling or one of the wall faces 41 is provided with an air conditioner 44 (an inhibitor supplier). The air conditioner 44 is a fixture, and capable of generating positive and negative ions as well as typically capable of conditioning air. This air conditioner 44 includes: an HEPA 45; an air blower 46; and an ion generator 47. Similar to the ion generator 8, the ion generator 47 generates positive and negative ions. The generated positive and negative ions flow on an airflow, generated by the air blower 46, to be released in the work space 48. The installation position and the airflow direction of the air conditioner 44 may be set so that the airflow to be generated by the air blower 46 travels throughout the work space 48.

The ion generator 47 supplies the positive and negative ions so that each of the positive ion concentration and the negative ion concentration in the work space 48 ranges preferably from 7,000 ions/cm³ to 1,000,000 ions/cm³, and more preferably from 7,000 ions/cm³ to 400,000 ions/cm³. The positive ion concentration and the negative ion concentration of 7,000 ions/cm³ or higher make it possible to curb the growth and proliferation of bacteria and viruses. Meanwhile, excessive concentrations of the positive ions and the negative ions might pose a problem for the cell culturing. Hence, each of the positive ion concentration and the negative ion concentration is preferably 1,000,000 ions/cm³ or lower, and more preferably 400,000 ions/cm³ or lower.

Note that upper limits of the positive ion concentration and the negative ion concentration vary, depending on cells to be cultured. Hence, the concentrations of the positive ions and the negative ions in the work space 48 may be set prior to the start of the cell culturing, depending on a kind of the cells to be cultured.

Examples of techniques to change the concentrations of the positive ions and the negative ions in the work space 48 include: (1) changing the number of the air conditioners 44 to be installed; (2) changing the number of operating air conditioners 44 (or operating ion generators 47) among the installed air conditioners 44 (or the installed ion generators 47), (3) changing the position of the air conditioner 44 to be installed, and (4) changing the velocity, volume, or direction of the airflow from the air blower 46. In order to make these adjustments possible, the air conditioner 44 may be provided as a movable device instead of a fixture.

The control device 50, which controls the air conditioner 44, may be provided inside or outside the work space 40, or installed in the air conditioner 44. The control device 50 may control the air conditioners 44 each provided to a corresponding one of the workrooms 40.

The control device 50 controls operations of the air conditioner 44 in accordance with a command of a user using the workroom 40. In particular, the control device 50 may adjust, in accordance with a command of the user, an amount of the positive and negative ions to be generated by the ion generator 47. The ion generator 47 may continuously generate the positive and negative ions. Note that the control device 50 may cause the ion generator 47 to keep from generating the positive and negative ions in a time frame when no asepsis is conducted (e.g., at night).

Moreover, the control device 50 may change the amount of the positive and negative ions to be supplied by the ion generator 47, depending on opening and closing of the door 43. For example, the control device 50 may increase the amount of the positive and negative ions to be supplied by the ion generator 47 for a predetermined time period (e.g., for five minutes) since the door 43 opens. Such a feature makes it possible to enhance advantageous effects of sterilization by the positive and negative ions in a situation in which contamination is likely to occur. In contrast, the control device 50 may decrease the amount of the positive and negative ions to be supplied by the ion generator 47 when the door 43 is neither opened nor closed for a long time.

The opening and closing of the door 43 is detected by a sensor 51 communicably connected to the control device 50. The sensor 51 may be a device to physically detect the opening and closing of the door 43, or a device, such as an infrared sensor, to optically detect the opening and closing of the door 43.

### Advantageous Effects of Cell Culturing System 60

In the cell culturing system 60, the ion generator 47 generates the positive ions and the negative ions to supply the ions to the work space 48, so that the supplied ions can deactivate the viruses present in the work space 48, and curb growth and proliferation of bacteria and molds found inside the work space 48.

Moreover, the insides of the safety cabinet 1, the clean chamber 20, and the incubator 30 provided in the work space 48 are sterilized by the positive and negative ions, making it possible to conduct sterilization more reliably.

The positive and negative ions cause no harm to humans and cultured cells, making it possible to sterilize the work space 48 while conducting work on cell culturing. Hence, the work on the cell culturing is not interrupted for the sterilization. Such a feature makes it possible to culture cells efficiently.

Moreover, a conventional sterilization technique using, for example, formalin cannot prevent contamination after the sterilization. However, the sterilization technique of the cell culturing system 60 can continuously conduct sterilization during the work on cell culturing. Hence, even if bacteria adhere to the worker, the cell culturing system 60 can curb growth and proliferation of the bacteria.

The cells cultured (produced) inside the work space 48 are also included in the technical scope of the present invention.

### Fifth Embodiment

In the above first to fourth embodiments, a substance other than the positive and negative ions can be used as a microorganism growth inhibitor.

The microorganism growth inhibitor to be used may include electric charges and low-temperature plasma (a second microorganism growth inhibitor). In this case, the safety cabinet 1, the clean chamber 20, the incubator 30, and the air conditioner 44 include an ionizer and an electric discharger as an inhibitor supplier, instead of the ion generators 8, 35, and 47. In this configuration, the electric discharger is installed in the ionizer. Provided downstream of the ionizer (downstream of an airflow) are an electrostatic filter and a catalytic filter.

The ionizer includes a plurality of ionization lines and a plurality of counter electrodes to charge relatively small dust, bacteria, and viruses (hereinafter "bacteria") in the air. These ionization lines and counter electrodes are positioned on an imaginary plane in parallel with the above electrostatic filter. The bacteria charged positively by the ionizer are collected with the electrostatic filter charged negatively.

The electric discharger, which generates low-temperature plasma, includes a discharge electrode and a counter electrode. The counter electrode included in this electric discharger is shared with the ionizer. Between the discharge electrode and the counter electrode, a discharge voltage is applied. The electric discharger supplies the low-temperature plasma toward the catalytic filter positioned downstream.

The low-temperature plasma to be generated by the electric discharger includes highly reactive substances, namely, such active species as electrons, ions, ozone, and radical. When these highly reactive substances reach the catalytic filter, the substances are activated further such that harmful substances and odoriferous substances are decomposed and removed.

The microorganism growth inhibitor to be used may include charged microparticles (the first microorganism growth inhibitor) in nanometer level including radical. The charged microparticles are a microorganism growth inhibitor causing no harm to humans. In this case, the safety cabinet 1 includes as an inhibitor supplier an electrostatic atomizer to generate the charged microparticles, instead of the ion generators 8, 35, and 47. When the electric field strength ranges from 700 to 1,200 V/mm, the mist generated by the electrostatic atomization is a group of namoparticles in nanometer level having a particle size ranging from 3 to 100 nm. These nanoparticles include radical (e.g., hydroxyl radical and superoxide) and have a large amount of electric charges.

The charged microparticles are supplied into the work spaces 3 and 48 or the incubator 30, making it possible to maintain the insides of the work spaces 3 and 48 and the incubator 30 clean.

The microorganism growth inhibitor to be used may include a chlorine compound (the second microorganism growth inhibitor). In this case, the safety cabinet 1 includes, instead of the ion generators 8, 35, and 47: a chlorine compound supplier (an inhibitor supplier) supplying a chlorine compound in the air; and a gas processing filter reacting to the chlorine compound. The gas processing filter includes fibers holding a compound having a primary amino group, ammonia, or ammonium salt. The gas processing filter is disposed more downstream of an airflow generated by the air blowers 9, 34, and 46 than the chlorine compound supplier is.

The chlorine compound supplier may include: a combination of a water storage tank storing chlorine-compound-containing water and a sprayer spraying the chlorine-compound-containing water; or a combination of the water storage tank and a vaporization accelerator improving an efficiency in gas-liquid contact to accelerate vaporization of the chlorine-compound-containing water. The vaporization accelerator is, for example, a body of water-absorbing fibers branched numerously.

Moreover, the chlorine compound supplier may further include an electrolytic unit electrolyzing chloride-ion-containing water to generate the chlorine-compound-containing water. The electrolytic unit includes an electrode plate to energize water containing chloride ion, namely, for example, water in which sodium chloride is dissolved, thereby generating water (electrolyzed water) containing a chlorine compound represented by hypochlorous acid. The generated chlorine-compound-containing water is stored in the storage tank.

The chlorine compound supplied by the chlorine compound supplier reacts to any one of the compound having the primary amino group, ammonia, or ammonium salt on the gas processing filter, and becomes combined chlorine. Hence, the chlorine compound supplier also supplies the chlorine compound to the gas processing filter.

When the airflow generated by the air blowers 9, 34, and 46 passes through the gas processing filter, bacteria contained in the airflow make contact with the combined chlorine, making it possible to curb growth and proliferation of the bacteria.

FIG. 6 is a diagram illustrating a positional relationship between the inhibitor supplier and a filter allowing passage of the gas to be supplied into the work space 3, in a state where the second microorganism growth inhibitor is supplied to the filter. As illustrated in FIG. 6, in the state where the second microorganism growth inhibitor is used, various kinds of the above-described filters (collectively referred to as a filter 70), which react to the second microorganism growth inhibitor, are disposed downstream of an inhibitor supplier 80 generating the second microorganism growth inhibitor. This configuration is applicable to the safety cabinet 1, the clean chamber 20, the incubator 30, and the air conditioner 44 described above.

As can be seen, the inhibitor supplier may supply a microorganism growth inhibitor into the culturing space 38 for culturing cells, or the work spaces 3 and 48. Moreover, the inhibitor supplier may supply a microorganism growth inhibitor to a filter allowing passage of the air (gas) to be supplied into the culturing space 38, or the work spaces 3 and 48.

### First Example

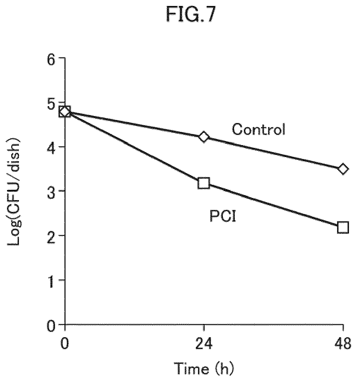

Described below are advantageous effects of the positive and negative ions, with reference to FIG. 7. In this example, a study was conducted to find out advantageous effects of the positive and negative ions on mycoplasmas (Mycoplasma pneumoniae NBRC14401).

A liquid nutrient medium of 10 µl in which the mycoplasmas grew was dispensed to ager plates (12 plates in total). Six of the ager plates were supplied with the positive and negative ions, and the rest of the ager plates were used as controls. The positive and negative ions were supplied into an acrylic box disposed in a safety cabinet for 24 hours or for 48 hours by an ion generation element (A272AK) manufactured by Sharp Corporation. The ion generation element was provided with an input voltage of DC 5V to cause an electric discharge and generate the ions.

Then, to form colonies, the total of 12 ager plates were placed in a jar containing AnaeroPack (produced by Mitsubishi Gas Chemical), and bacteria were cultured for five days at a temperature of 37 °C After the culturing, the number of colonies were counted, using a microscope of 40 times power. FIG. 7 shows the result.

As illustrated in FIG. 7, it is clear that the number of growing mycoplasmas was significantly smaller (p < 0.002) when the positive and negative ions (PCI) were provided than when no PCI was provided.

As a second example to be described later shows in an example, it has already been evident that the positive and negative ions have advantageous effects to curb growth and proliferation of various kinds of bacteria and to deactivate viruses. Moreover, it has also become evident that the advantageous effects depend on the concentrations of the positive and negative ions. Hence, the advantageous effects of the positive and negative ions on the mycoplasmas are considered to be dependent on the concentrations.

In this example, the positive ion concentration and the negative ion concentration around the ager plates are approximately 7,000 ions/cm³. Hence, the positive-and-negative-ion concentrations as high as those necessary to curb growth and proliferation of other bacteria are considered to be able to curb growth and proliferation of mycoplasmas.

Mycoplasmas are a main cause of contamination when cells are cultivated for regenerative medicine. Mycoplasmas are presumed to be derived from the body of a worker, such that the contamination occurs when the cells are subcultured. A typical sterilization technique has difficulty in prevention of such contamination.

The positive and negative ions cause no harm to humans and cultured cells, and can be provided during work. Providing such positive and negative ions makes it possible to prevent contamination by mycoplasmas, contributing to efficiently culturing cells for regenerative medicine.

### Second Example

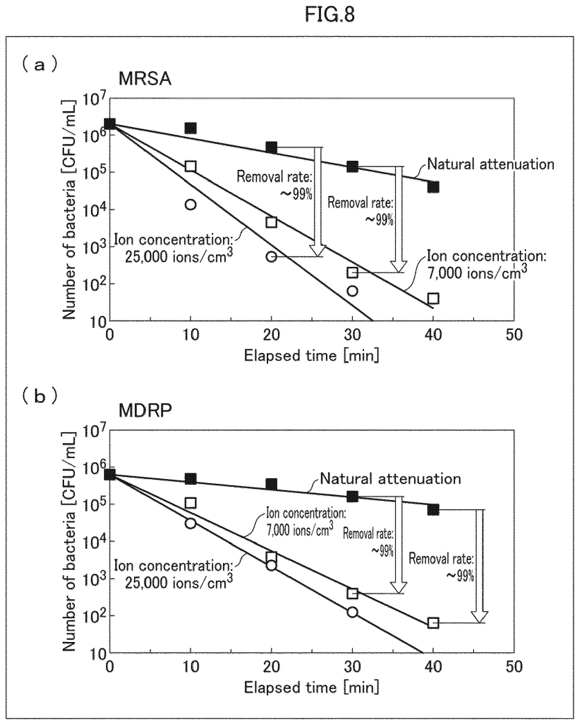

In this example, a study was conducted to find out advantageous effects of the positive and negative ions on airborne bacteria. FIG. 8 is a graph illustrating advantageous effects of the positive and negative ions on airborne bacteria. The airborne bacteria to be removed are methicillin-resistant Staphylococcus aureus (MRSA) and multidrug-resistant Pseudomonas aeruginosa (MDRP).

An ion generator manufactured by manufactured by Sharp Corporation was disposed in a chamber whose internal space has a volumetric capacity of one cubic meter, and a fungus liquid containing MRSA and MDRP was sprayed into the chamber. Positive and negative ions were generated by the ion generator. Then, in 0 minutes, 10 minutes, 20 minutes, 30 minutes, 40 minutes, and 50 minutes, the airborne bacteria in the chamber were collected with an impinge, and the number of the bacteria in the collected liquid was evaluated.

As shown in an illustration (a) in FIG. 8, approximately 99.9% of MRSA was removed in 30 minutes when the positive and negative ions having a concentration of 7,000 ions/cm³ were generated, compared with a case of natural attenuation. Approximately 99.9% of MRSA was removed in 20 minutes when the positive and negative ions having a concentration of 25,000 ions/cm³ were generated.

As shown in an illustration (b) in FIG. 8, approximately 99.9% of MDRP was removed in 40 minutes when the positive and negative ions having a concentration of 7,000 ions/cm³ were generated, compared with a case of natural attenuation. Approximately 99.9% of MDRP was removed in 30 minutes when the positive and negative ions having a concentration of 25,000 ions/cm³ were generated.

The above results clearly show that the positive and negative ions have advantageous effects to curb growth and proliferation of the MRSA and the MDRP, and the advantageous effects are dependent on the concentrations of the positive and negative ions. Hence, the advantageous effects of the positive and negative ions on the mycoplasmas described in the first example are considered to be dependent also on the concentrations of the positive and negative ions.

### Summary

A clean space maintenance device according to a first aspect of the present invention includes: a space definer defining a space for culturing cells or conducting work on the culturing; and an inhibitor supplier supplying a first microorganism growth inhibitor to the space, or a second microorganism growth inhibitor to a filter allowing passage of gas to be supplied to the space, the first microorganism growth inhibitor causing no harm to humans.

The above features allow the space to be sterilized without affecting humans and cultured cells, making it possible to continuously culture the cells without interruption by the sterilization.

A second aspect of the present invention is directed to the clean space maintenance device according to the first aspect, wherein the inhibitor supplier may generate the first microorganism growth inhibitor or the second microorganism growth inhibitor by electric discharge or electrolysis.

A third aspect of the present invention is directed to the clean space maintenance device according the first or second aspect, wherein the first microorganism growth inhibitor may include positive ions and negative ions.

The positive and negative ions act as a microorganism growth inhibitor, making it possible to effectively sterilize the work space, without affecting humans and cells to be cultured.

A fourth aspect of the present invention is directed to the clean space maintenance device according to the third aspect, wherein the inhibitor supplier may supply the positive ions and the negative ions so that a positive ion concentration of the positive ions and a negative ion concentration of the negative ions in the space may be each 7,000 ions/cm³ or higher.

A fifth aspect of the present invention is directed to the clean space maintenance device according to the fourth aspect, wherein the inhibitor supplier may supply the positive ions and the negative ions so that the positive ion concentration and the negative ion concentration in the space each range from 7,000 ions/cm³ to 1,000,000 ions/cm³.

In the above features, the positive ion concentration and the negative ion concentration of 7,000 ions/cm³ or higher make it possible to curb growth and proliferation of bacteria. Moreover, the positive ion concentration and the negative ion concentration of 1,000,000 ions/cm³ or lower make it possible to reduce adverse effects on cells to be cultivated.

A cell culturing method according to a sixth aspect of the present invention includes steps of: (a) supplying a first microorganism growth inhibitor to a space for culturing cells or conducting work on the culturing, or clean air to the space, the first microorganism growth inhibitor causing no harm to humans, and the clean air being generated using a second microorganism growth inhibitor; and (b) culturing cells or conducting work on the culturing inside the space supplied with the first microorganism growth inhibitor, or with the clean air.

In the cell culturing method according to a seventh aspect of the present invention, the first microorganism growth inhibitor or the second microorganism growth inhibitor may have a concentration set, depending on a kind of the cells to be cultured.

In the cell culturing method according to eighth aspect of the present invention, the cells to be cultured may include induced pluripotent stem cells.

A cell production method according to a ninth aspect of the present invention includes steps of: (a) supplying a first microorganism growth inhibitor to a space for culturing cells or conducting work on the culturing, or clean air to the space, the first microorganism growth inhibitor causing no harm to humans, and the clean air being generated using a second microorganism growth inhibitor; and (b) culturing cells or conducting work on the culturing inside the space supplied with the first microorganism growth inhibitor, or with the clean air, thereby producing cultured cells.

A building structure according to a tenth aspect of the present invention defines a space for culturing cells or conducting work on the culturing. The building structure includes an inhibitor supplier supplying a first microorganism growth inhibitor to the space, or a second microorganism growth inhibitor to a filter allowing passage of gas to be supplied to the space, the first microorganism growth inhibitor causing no harm to humans.

The eleventh aspect of the present invention is directed to the building structure according to the tenth aspect. The building structure may further include a door to enter and exit the space, wherein
the inhibitor supplier may change an amount of the first microorganism growth inhibitor to be supplied, depending on opening and closing of the door.

The above features make it possible to adjust advantageous effects of sterilization achieved by the first microorganism growth inhibitor, depending on a situation in which contamination is likely to occur; that is, whether the door is opened or closed.

### Reference Signs List

- 1: Safety Cabinet
- 3: Work Space
- 4: Space Definer
- 8, 35, 47: Ion Generator (Inhibitor Supplier)
- 20: Clean Chamber
- 30: Incubator
- 38: Culturing Space
- 40: Workroom
- 43: Door
- 44: Air Conditioner
- 48: Work Space
- 50: Control Device
- 60: Cell Culturing System
- 70: Filter
- 80: Inhibitor Supplier

## Claims

1. A clean space maintenance device, comprising:
a space definer defining a space for culturing cells or conducting work on the culturing; and
an inhibitor supplier configured to supply a first microorganism growth inhibitor to the space, or a second microorganism growth inhibitor to a filter allowing passage of gas to be supplied to the space, the first microorganism growth inhibitor causing no harm to humans.

2. The clean space maintenance device according to claim 1, wherein
the inhibitor supplier generates the first microorganism growth inhibitor or the second microorganism growth inhibitor by electric discharge or electrolysis.

3. The clean space maintenance device according to claim 1 or 2, wherein
the first microorganism growth inhibitor includes positive ions and negative ions.

4. The clean space maintenance device according to claim 3, wherein
the inhibitor supplier supplies the positive ions and the negative ions so that a positive ion concentration of the positive ions and a negative ion concentration of the negative ions in the space are each 7,000 ions/cm³ or higher.

5. The clean space maintenance device according to claim 4, wherein
the inhibitor supplier supplies the positive ions and the negative ions so that the positive ion concentration and the negative ion concentration in the space each range from 7,000 ions/cm³ to 1,000,000 ions/cm³.

6. A cell culturing method, comprising steps of:
(a) supplying a first microorganism growth inhibitor to a space for culturing cells or conducting work on the culturing, or clean air to the space, the first microorganism growth inhibitor causing no harm to humans, and the clean air being generated using a second microorganism growth inhibitor; and
(b) culturing cells or conducting work on the culturing inside the space supplied with the first microorganism growth inhibitor, or with the clean air.

7. The cell culturing method according to claim 6, wherein
the first microorganism growth inhibitor or the second microorganism growth inhibitor has a concentration set, depending on a kind of the cells to be cultured.

8. The cell culturing method according to claim 6 or 7, wherein
the cells to be cultured include induced pluripotent stem cells.

9. A cell production method, comprising steps of:
(a) supplying a first microorganism growth inhibitor to a space for culturing cells or conducting work on the culturing, or clean air to the space, the first microorganism growth inhibitor causing no harm to humans, and the clean air being generated using a second microorganism growth inhibitor; and
(b) culturing cells or conducting work on the culturing inside the space supplied with the first microorganism growth inhibitor, or with the clean air, thereby producing cultured cells.

10. A building structure which defines a space for culturing cells or conducting work on the culturing, the building structure comprising
an inhibitor supplier configured to supply a first microorganism growth inhibitor to the space, or a second microorganism growth inhibitor to a filter allowing passage of gas to be supplied to the space, the first microorganism growth inhibitor causing no harm to humans.

11. The building structure according to claim 10, further comprising
a door to enter and exit the space, wherein
the inhibitor supplier changes an amount of the first microorganism growth inhibitor to be supplied, depending on opening and closing of the door.
